# EUROPEAN PATENT APPLICATION

(11) **EP 4 752 128 A1**
(43) Date of publication of application: **03.06.2026**
(21) Application number: 23946719.4
(22) Date of filing: 27.07.2023
(51) Int. Cl.: C07C 45/66, C07C 67/00, C07D 231/14, C07C 69/74

(54) **METHOD FOR PRODUCING CYCLIZED PRODUCT BY CYCLIZATION REACTION INVOLVING DEHYDRATION CONDENSATION OF CARBOXYLIC ACID ESTER DERIVATIVE, AND METHOD FOR PRODUCING 1,3,4-SUBSTITUTED-PYRAZOLE-5-CARBOXYLIC ACID ESTER**

(71) Applicant: Mitsubishi Gas Chemical Next Company, Inc., Tokyo, 105-7116 (JP)
(72) Inventor: KURODA, Toshihiro, Niigata-shi, Niigata 950-3126 (JP); IIJIMA, Daiki, Niigata-shi, Niigata 950-3126 (JP); WASUZU, Ayase, Niigata-shi, Niigata 950-3126 (JP)
(74) Representative: Gille Hrabal Partnerschaftsgesellschaft mbB Patentanwälte
(86) International application number: PCT/JP2023/027548
(87) International publication number: WO 2025/022637

(57) **Abstract**

The invention relates to a reaction method including using a carbonic acid ester compound as a dehydration agent in a cyclization reaction involving dehydration condensation of a carboxylic acid ester derivative, and specifically, the invention relates to an industrially usable method for producing a 1,3,4-substituted pyrazole-5-carboxylic acid ester compound, including using a carbonic acid ester compound as a dehydration agent in a reaction of cyclizing a hydrazinoacetic acid ester derivative.

## Description

### Technical Field

The present invention relates to a production method for producing a cyclized product, such as a 1,3,4-substituted pyrazole-5-carboxylic acid ester compound, through a cyclization reaction involving dehydration condensation of a carboxylic acid ester derivative, by a novel reaction method capable of synthesizing the target product with a high yield without necessity of removing by-product water, and specifically relates to an industrial method using the production method, for producing a 1,3,4-substituted pyrazole-5-carboxylic acid ester compound, which is useful as an important intermediate in the field of medical and agricultural chemicals and the like.

### Background Art

As shown in PTLs 1 and 2, a 1,3,4-substituted pyrazole-5-carboxylic acid ester compound has been produced by a method of chloromethylating the 4-position of a 1,3-dimethylpyrazole-5-carboxylic acid ester, and then introducing a methyl group thereto through dechlorination under reducing condition. However, the method has problems including the formation of bis(chloromethyl) ether, which is a harmful substance, in the chloromethylation reaction of the 4-position of a 1,3-dimethylpyrazole-5-carboxylic acid ester. PTL 3 synthesizes ethyl 1,3,4-trimethylpyrazole-5-carboxylate by reacting an intermediate synthesized with ethyl bromoacetate, dimethyl sulfide, and diacetyl, with monomethylhydrazine. However, the method causes a low yield of 10% due to the 4-step reaction thereof, and uses dimethyl sulfide having an objectionable odor.

PTL 4 describes a method for providing a 1,3,4-trimethylpyrazole-5-carboxylic acid ester compound through a cyclization reaction of a hydrazinoacetic acid ester derivative in the presence of a base. In the reaction, the hydrazinoacetic acid ester derivative is subjected to azeotropic dehydration, and then reacted. As a dehydration method other than the azeotropic dehydration, the addition of an inorganic solid dehydration agent, such as calcium chloride and molecular sieve, is described, but the dehydration agents are necessarily separated from the target product, for example, by filtration, after completing the reaction.

PTLs 5 and 6 use a carbonic acid ester compound in pyrazole synthesis. However, a cyanoalkene derivative or an alkoxymethylene compound is synthesized through a cyclization reaction with hydrazine, which is different in reaction mode from the pyrazole synthesis through a cyclization reaction of a hydrazinoacetic acid ester derivative in the present invention, and the literatures have no description about the effectiveness of a carbonic acid ester compound as a dehydration agent.

### Citation List

### Patent Literatures

PTL 1: JP 2001-342178 A
PTL 2: JP 2008-007503 A
PTL 3: WO 2010/045764
PTL 4: JP 2008-208047 A
PTL 5: JP 2001-58982 A
PTL 6: JP 2000-212166 A

### Summary of Invention

### Technical Problem

As described above, the known production methods of a 1,3,4-substituted pyrazole-5-carboxylic acid ester compound cannot necessarily be said to be a method that is industrially satisfactory, due to the necessity of the dehydration process from the liquid phase caused by the azeotropic dehydration and the filtering process caused by the use of the inorganic solid dehydration agent, and also due to the yield of the target product. Under the circumstances, there has been a demand of a production method of the compound that uses a dehydration agent that does not necessitate a complicated post-process.

In consideration of the circumstances, an object of the present invention is to provide a method for producing a cyclized product based on a novel reaction that does not necessitate azeotropic dehydration and a filtering process for an inorganic solid dehydration agent, and exhibits a high yield, and to provide, as one of embodiment of the production method, a 1,3,4-substituted pyrazole-5-carboxylic acid ester compound, which is useful as a medical and agricultural intermediate, through a cyclization reaction of a hydrazinocarboxylic acid ester derivative.

### Solution to Problem

As a result of the earnest investigations for solving the problem by the present inventors, it has been found that in a cyclization reaction involving dehydration condensation, such as providing a 1,3,4-substituted pyrazole-5-carboxylic acid ester through cyclization of a hydrazinoacetic acid ester derivative, the use of a carbonic acid ester compound in the presence of a base allows the carbonic acid ester compound to function as a dehydration agent, so as to achieve a reaction method that does not necessitate the azeotropic dehydration for removing by-product water generated in the dehydration condensation reaction for enhancing the yield, and the filtering process for the inorganic solid dehydration agent used, and it has actually been revealed that in providing a 1,3,4-substituted pyrazole-5-carboxylic acid ester through dehydration cyclization of a hydrazinoacetic acid ester derivative, the use of a carbonic acid ester compound as like as a solvent can provide a 1,3,4-substituted pyrazole-5-carboxylic acid ester with a high yield even in the state where by-product water is mixed in the reaction system.

In summary, it has been found in the present invention that in the cyclization reaction involving dehydration condensation of a carboxylic acid ester derivative, a carbonic acid ester compound used functions as a dehydration agent, and for example, also found that a 1,3,4-substituted pyrazole-5-carboxylic acid ester compound, which is a cyclized product obtained from a hydrazinoacetic acid ester derivative, can be conveniently produced in an industrial scale with no necessity of a complicated post-process, and thus the present invention has been completed.

Specifically, the present invention relates to a method for producing a cyclized product, including using a carbonic acid ester compound as a dehydration agent, in a cyclization reaction involving dehydration condensation of a carboxylic acid ester derivative, and also relates to a method for producing a pyrazole carboxylic acid ester compound, including using a hydrazinocarboxylic acid derivative as the carboxylic acid ester derivative, and forming a pyrazole ring through a cyclization reaction involving dehydration condensation.

More specifically, the present invention relates to a method for producing a 1,3,4-substituted pyrazole-5-carboxylic acid ester compound represented by the general formula (2): 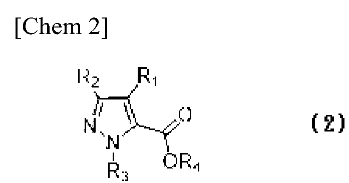 in which R₁ to R₃ each represents a hydrogen atom or an alkyl group having 1 to 4 carbon atoms, and R₄ represents an alkyl group having 1 to 4 carbon atoms, including reacting a carbonic acid ester compound as a dehydration agent, preferably in the presence of a base, with a hydrazinoacetic acid ester derivative represented by the general formula (1): 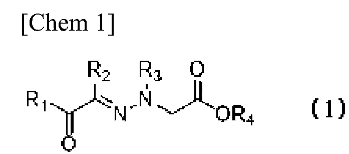 in which R₁ to R₄ have the same meanings as above.

### Advantageous Effects of Invention

The present invention provides an industrial production method of a cyclized product capable of providing the cyclized product with a high yield that does not necessitate the azeotropic dehydration for enhancing the yield, the use of an inorganic solid dehydration agent, and the filtering process therefor, by using a carbonic acid ester compound in the cyclization reaction involving dehydration condensation of a carboxylic acid ester derivative, and the present invention also provides a production method for producing a 1,3,4-substituted pyrazole-5-carboxylic acid ester compound through cyclization reaction involving dehydration condensation from a hydrazinoacetic acid ester derivative, in which a carbonic acid ester compound used as a dehydration agent functions as an effective dehydration agent for water by-produced in the reaction, and obviates the need for the azeotropic dehydration process and the filtering process for an inorganic solid dehydration agent, and thereby the cyclized product can be industrially produced more advantageously.

### Description of Embodiments

The production methods of the present invention are described in more detail below.

The present invention is an invention relating to a method for producing a cyclized product through cyclization reaction involving dehydration condensation of a carboxylic acid ester derivative, including using a carbonic acid ester compound as a dehydration agent.

The cyclization reaction that the present invention targets involves dehydration condensation, and for example, is a reaction shown by the reaction formula (A) described later, in which a hydrazinoacetic acid ester derivative (1), which is the carboxylic acid ester derivative, is used, and in the presence of a base, enolate formed by withdrawing hydrogen at the α-position of the acetic acid ester attacks carbon of the carbonyl group, so as to perform intramolecular cyclization with by-production of water, and reaction examples of the intramolecular cyclization reaction also include an intramolecular aldol reaction.

The "cyclized product" referred in the present invention is a compound that is obtained through cyclization reaction involving dehydration condensation of a carboxylic acid ester derivative, which is a compound having a carboxylic acid ester and a ring structure, in which a ring is formed through intramolecular cyclization, and the carboxylic acid ester in the starting material remains, and is generally a production target compound.

Examples of the carbonic acid ester compound utilized as a dehydration agent include dimethyl carbonate, diethyl carbonate, dimethyl dicarbonate, diethyl dicarbonate, and di-tert-butyl dicarbonate, and these carbonic acid ester compounds each may be used alone, or two or more kinds thereof may be used in combination.

In the reaction method, the carbonic acid ester compound can be used as like as a solvent, and it suffices that the carbonic acid ester compound is added with a solvent in the case where a solvent is used, or is added alone in the case where a solvent is not used, to the reaction system along with the starting material, which are subjected to the reaction as they are, and thereby the cyclized product can be produced by a convenient reaction method that does not necessitate an operation of removing by-product water generated in the dehydration condensation reaction, such as a dehydration operation through azeotropic dehydration or the like and the use and removal of an inorganic solid dehydration agent.

In the reaction, a solvent and a catalyst may be used depending on necessity, and the charge amounts of the starting material including the solvent and the catalyst, and the carbonic acid ester compound, and the reaction condition including the reaction temperature, the reaction pressure, the reaction time, and the like are appropriately selected depending on the kind of the cyclized product to be produced through the reaction. In general, the carbonic acid ester compound can be used in a range of 0.5 to 10.0 equivalents per 1 equivalent of the starting material, and the reaction can be performed under condition of a reaction temperature of -10 to 130°C and a reaction time of approximately 1 to 15 hours.

The mechanism that the use of the carbonic acid ester compound enables the production of the cyclized product with a good yield without dehydration of by-product water is considered as follows: the carbonic acid ester compound undergoes hydrolysis extremely rapidly as compared to the starting material and the cyclized product, and therefore water is consumed through the preferential reaction between by-product water and the carbonic acid ester compound, so that the reaction is accelerated, and simultaneously the hydrolysis of the carboxylic acid ester is prevented. It is also considered that in the presence of a base, the carbonic acid ester compound is reacted with water to become a stable carbonate salt, and thereby side reactions, such as hydrolysis of the starting material and the cyclized product, do not proceed. These phenomena are similarly observed in the reaction of providing a 1,3,4-substituted pyrazole-5-carboxylic acid ester compound through intramolecular cyclization caused by dehydration condensation of a hydrazinoacetic acid ester derivative described below, and also observed in reactions with the similar reaction mechanism, i.e., other formation reactions of cyclized products through intramolecular cyclization reaction forming by-product water by dehydration condensation of a carboxylic acid ester derivative.

In the method for producing a cyclized product using a carboxylic acid ester derivative, examples of the method for producing a cyclized product through intramolecular cyclization reaction using another starting material include the following. 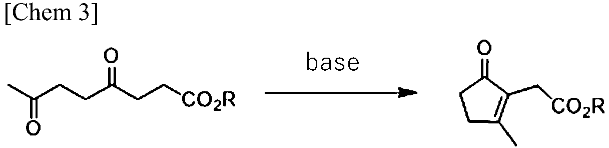 in which R represents an alkyl group having 1 to 4 carbon atoms.

A specific method for producing a 1,3,4-substituted pyrazole-5-carboxylic acid ester according to the present invention is described below. The production method produces a 1,3,4-substituted pyrazole-5-carboxylic acid ester compound represented by the general formula (2) by using a carbonic acid ester compound as a dehydration agent in the cyclization reaction of the hydrazinoacetic acid ester derivative (1). The reaction process is shown by the following reaction formula (A). 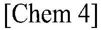 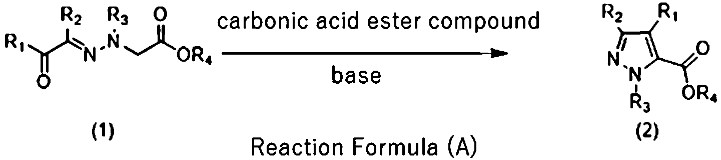 In the compounds represented by the formulae (1) and (2), the substituents R₁ to R₃ each represent a hydrogen atom or an alkyl group having 1 to 4 carbon atoms, and R₄ represents an alkyl group having 1 to 4 carbon atoms. Examples of the alkyl group having 1 to 4 carbon atoms generally include a methyl group, an ethyl group, a n-propyl group, an isopropyl group, a n-butyl group, an isobutyl group, a sec-butyl group, and a tert-butyl group.

The production method of the present invention can be performed by reacting the hydrazinoacetic acid ester derivative in the presence of a base and the carbonic acid ester compound as a dehydration agent for a prescribed period of time at a prescribed temperature and a prescribed pressure.

Examples of the carbonic acid ester compound that can be used as a dehydration agent include dimethyl carbonate, diethyl carbonate, dimethyl dicarbonate, diethyl dicarbonate, and di-tert-butyl dicarbonate, and these compounds each may be used alone, or two or more kinds thereof may be used in combination. Among these, a carbonic acid ester compound is preferred, and dimethyl carbonate and diethyl carbonate are particularly preferred. The amount of the dehydration agent used is preferably 0.5 to 10.0 equivalents, and particularly preferably 1 to 3 equivalents, based on the hydrazinoacetic acid ester derivative (1) as the substrate.

Examples of the base that can be used in the reaction and functions as a so-called catalyst include an alkali metal carbonate compound, such as potassium carbonate and sodium carbonate, a metal alkoxide compound, such as sodium methoxide, sodium ethoxide, and potassium tert-butoxide, and an organic base compound, such as pyridine, morpholine, and triethylamine. These bases each may be used alone, or two or more kinds thereof may be used in combination. Among these, a metal alkoxide compound is preferred, and sodium methoxide and sodium ethoxide are particularly preferred. The metal alkoxide compound may be used in the form of an alcohol solution or in the form of powder thereof itself. The amount of the base used is preferably 0.5 to 5 equivalents, and particularly preferably 1.0 to 1.5 equivalents, based on the hydrazinoacetic acid ester derivative (1) as the substrate.

The reaction temperature is preferably -10 to 130°C, and more preferably 30 to 50°C.

The reaction time is preferably 1 to 5 hours, and more preferably 2 to 3 hours. In the case where the condition is in the ranges, the 1,3,4-substituted pyrazole-5-carboxylic acid ester can be obtained with a high yield of 80% or more.

The reaction pressure is preferably ordinary pressure from the standpoint of the handleability, or may be reduced pressure or increased pressure in consideration of the vapor pressure of the reaction system and the reaction temperature.

A solvent may be used as necessary from the standpoint of the solubility of the starting material and the cyclized product and the reactivity. The solvent used is generally preferably an aliphatic hydrocarbon based solvent, an alicyclic hydrocarbon based solvent, an aromatic based solvent, an alcohol based solvent, an ester based solvent, and the like while depending on the kinds of the starting material and the cyclized product, and one kind of the solvent may be used alone, or two or more kinds thereof may be used in combination. Specifically, methanol, ethanol, and the like are preferably used in consideration of dissolution of the base. The amount of the solvent blended is preferably 50 to 100 parts by mass, and more preferably approximately 80 parts by mass, per 100 parts by mass of the hydrazinoacetic acid ester derivative (1).

After completing the reaction, the reaction liquid may be treated by the known method, such as neutralization and washing-separation, and then the 1,3,4-substituted pyrazole-5-carboxylic acid ester compound can be isolated by the known method, such as distillation and crystallization-separation.

In the neutralization, the washing-separation, and the like, a solvent may be added to the reaction liquid after completing the reaction, and examples of the usable solvent include the aforementioned solvents that can be used in the reaction. In consideration of the efficiency in the washing-separation and the like, a solvent that is separated from water is preferred, and the amount of the solvent is preferably approximately 150 parts by mass per 100 parts by mass of the resulting 1,3,4-substituted pyrazole-5-carboxylic acid ester compound.

The distillation and the crystallization-separation may be performed according to the known method, and in the distillation, for example, the set condition may be selected depending on the properties of the target product, in which while not particularly limited, it is preferred that an apparatus equipped with a distillation tower is used, in which the heating temperature is preferably 30 to 300°C, and more preferably 50 to 180°C, and the pressure is preferably ordinary pressure of 760 torr to high vacuum of 0.1 torr, and more preferably 500 torr to 1 torr. The number of steps of the distillation tower is preferably 5 to 40 steps, and more preferably 10 to 30 steps. The reflux ratio is preferably 1 to 40, and more preferably 2 to 20. In the case where crystals are deposited in cooling, concentrating, or the like of the treated reaction liquid, the deposited crystals can be isolated by separating with a centrifugal separator or the like. The temperature in the separation is preferably 40 to -10°C, and more preferably 30 to -5°C. In the concentrating condition, the heating temperature is preferably 30 to 300°C, and the pressure is preferably ordinary pressure of 760 torr to 10 torr.

The 1,3,4-substituted pyrazole-5-carboxylic acid ester compound in the present embodiment can be generally distillated at approximately 40 to 3 torr and approximately 80 to 140°C while depending on the kind of the ester.

In the treatment after completing the reaction described above, a concentrating step may be provided preceding the washing in the process including concentrating after neutralization, and then washing, and thereby the product yield can be enhanced by 3 to 5%, from which it has been found that one of the preferred production methods can be obtained by providing the concentrating step for providing the valuable cyclized product.

The hydrazinoacetic acid ester derivative represented by the general formula (1) can be synthesized according to the method described in JP 2008-208047 A, in which a hydrazine compound and a halogenoacetic acid ester, such as bromoacetic acid ester, chloroacetic acid ester, or iodoacetic acid ester, are reacted, and then diacetyl is reacted therewith.

### Examples

The present invention and inventions as embodiments thereof are specifically described with reference to examples below, but the present invention is not limited in scope by the examples. In Examples and Comparative Examples below, the yield was calculated by using high-performance liquid chromatography (HPLC).

### Reference Example 1

### Synthesis of Methyl 2-[(1-Aza-2-methyl-3-oxobut-1-enyl)methylamino]acetate

In a 2 L four-neck flask, 149.7 g (3.25 mol) of monomethylhydrazine, 520.7 g (16.25 mol) of methanol, and 296.0 g (2.93 mol) of triethylamine were charged and cooled to 5°C under stirring. 497.2 g (3.25 mol) of methyl bromoacetate was added thereto in a dropwise manner over 2 hours. Subsequently, the reaction was performed for 1 hour, to which 265.8 g (3.09 mol) of diacetyl was added in a dropwise manner over 1.5 hours, and the reaction was performed at 10°C for 19 hours. The resulting reaction liquid was concentrated under reduced pressure, to which 900.0 g of toluene and 404.8 g of water were added, followed by stirring. After removing the aqueous layer through liquid separation, 147.2 g of water was added to the toluene layer for washing. After removing the aqueous layer through liquid separation, the toluene layer was concentrated under reduced pressure to provide 541.2 g of methyl 2-[(1-aza-2-methyl-3-oxobut-1-enyl)methylamino]acetate. The yield based on methyl bromoacetate used was 74.7%.

### Reference Example 2

### Synthesis of Ethyl 2-[(1-Aza-2-methyl-3-oxobut-1-enyl)methylamino]acetate

In a 3 L four-neck flask, 149.7 g (3.25 mol) of monomethylhydrazine, 748.6 g (16.25 mol) of ethanol, and 296.0 g (2.93 mol) of triethylamine were charged and cooled to 5°C under stirring. 542.8 g (3.25 mol) of ethyl bromoacetate was added thereto in a dropwise manner over 2 hours. Subsequently, the reaction was performed for 1 hour, to which 265.8 g (3.09 mol) of diacetyl was added in a dropwise manner over 1.5 hours, and the reaction was performed at 10°C for 19 hours. The resulting reaction liquid was concentrated under reduced pressure, to which 900.0 g of toluene and 404.8 g of water were added, followed by stirring. After removing the aqueous layer through liquid separation, 147.2 g of water was added to the toluene layer for washing. After removing the aqueous layer through liquid separation, the toluene layer was concentrated under reduced pressure to provide 551.2 g of ethyl 2-[(1-aza-2-methyl-3-oxobut-1-enyl)methylamino]acetate. The yield based on ethyl bromoacetate used was 72.0%.

### Example 1

### Synthesis of Methyl 1,3,4-Trimethylpyrazole-5-carboxylate using Dimethyl Carbonate as Dehydration Agent

In a 2 L four-neck flask, 247.7 g (1.28 mol) of a 28% sodium methoxide methanol solution and 192.8 g (2.15 mol) of dimethyl carbonate were charged and heated to 40°C under stirring. 229.4 g (1.07 mol) of methyl 2-[(1-aza-2-methyl-3-oxobut-1-enyl)methylamino]acetate was added thereto in a dropwise manner over 2.0 hours. Subsequently, the reaction was performed at 40°C for 2 hours. After completing the reaction, the reaction liquid was cooled to 20°C and neutralized with 129.3 g (1.24 mol) of hydrochloric acid. The neutralized liquid was concentrated at a reduced pressure of 10 kPa, to which 345.1 g of toluene and 264.8 g of water were added, followed by stirring. After removing the aqueous layer, the toluene layer was washed with water. The toluene layer, from which the washing water had been removed, contained 29.9% by weight of methyl 1,3,4-trimethylpyrazole-5-carboxylate. The yield based on methyl 2-[(1-aza-2-methyl-3-oxobut-1-enyl)methylamino]acetate used was 88.2%. The yield of 1,3,4-trimethylpyrazole-5-carboxylic acid as a hydrolysate was 5.3%. The yield in the case where the reaction liquid was neutralized with hydrochloric acid, and then washed without concentrating was 84.9%, from which it was found that the yield was enhanced by adding the concentrating step.

### Example 2

### Synthesis of Ethyl 1,3,4-Trimethylpyrazole-5-carboxylate using Diethyl Carbonate as Dehydration Agent

In a 3 L four-neck flask, 816.6 g (2.40 mol) of a 20% sodium ethoxide ethanol solution and 472.5 g (4.00 mol) of diethyl carbonate were charged and heated to 40°C under stirring. 479.3 g (2.00 mol) of ethyl 2-[(1-aza-2-methyl-3-oxobut-1-enyl)methylamino]acetate was added thereto in a dropwise manner over 2.5 hours. Subsequently, the reaction was performed at 40°C for 2 hours. After completing the reaction, the reaction liquid was cooled to 20°C and neutralized with 233.7 g (2.24 mol) of hydrochloric acid. 654.0 g of toluene and 504.3 g of water were added thereto, followed by stirring. After removing the aqueous layer, the toluene layer was washed with water. The toluene layer, from which the washing water had been removed, contained 17.4% by weight of ethyl 1,3,4-trimethylpyrazole-5-carboxylate. The yield based on ethyl 2-[(1-aza-2-methyl-3-oxobut-1-enyl)methylamino]acetate used was 85.3%. The yield of 1,3,4-trimethylpyrazole-5-carboxylic acid as a hydrolysate was 6.9%.

### Comparative Example 1

### Synthesis of Methyl 1,3,4-Trimethylpyrazole-5-carboxylate using No Dehydration Agent

In a 2 L four-neck flask, 247.7 g (1.28 mol) of a 28% sodium methoxide methanol solution was charged and heated to 40°C under stirring. 229.4 g (1.07 mol) of methyl 2-[(1-aza-2-methyl-3-oxobut-1-enyl)methylamino]acetate was added thereto in a dropwise manner over 2.0 hours. Subsequently, the reaction was performed at 40°C for 2 hours. After completing the reaction, the reaction liquid was cooled to 20°C and neutralized with 129.3 g (1.24 mol) of hydrochloric acid. 345.1 g of toluene and 264.8 g of water were added thereto, followed by stirring. After removing the aqueous layer, the toluene layer was washed with water. The toluene layer, from which the washing water had been removed, contained 11.5% by weight of methyl 1,3,4-trimethylpyrazole-5-carboxylate. The yield based on methyl 2-[(1-aza-2-methyl-3-oxobut-1-enyl)methylamino]acetate used was 25.8%. The yield of 1,3,4-trimethylpyrazole-5-carboxylic acid as a hydrolysate was 42.8%.

### Comparative Example 2

### Synthesis of Methyl 1,3,4-Trimethylpyrazole-5-carboxylate by performing Reaction after Dehydration in advance

To a 500 mL four-neck flask, 32.2 g (0.15 mol) of methyl 2-[(1-aza-2-methyl-3-oxobut-1-enyl)methylamino]acetate, 10.0 g (0.11 mol) of toluene, and 300.0 g (9.36 mol) of methanol were charged and heated under ordinary pressure. 31.3 g of a fraction was collected, and water in the system was removed. After cooling with ice, 20.7 g (0.15 mol) of potassium carbonate was placed therein, followed by heating under refluxing for 4 hours. After cooling with ice, methanol was recovered under reduced pressure, 100 g of water and 100 g of n-hexane were added, and the organic matter was extracted. The aqueous layer was further extracted with 30 g of n-hexane, and the extract was added to the organic layer. The organic layer contained 1.4% by weight of methyl 1,3,4-trimethylpyrazole-5-carboxylate. The yield based on methyl 2-[(1-aza-2-methyl-3-oxobut-1-enyl)methylamino]acetate used was 7.1%. The yield of 1,3,4-trimethylpyrazole-5-carboxylic acid as a hydrolysate was 70.1%.

### Comparative Example 3

In a 2 L four-neck flask, 247.7 g (1.28 mol) of a 28% sodium methoxide methanol solution and 227.1 g (2.14 mol) of methyl o-formate were charged and heated to 40°C under stirring. 229.4 g (1.07 mol) of methyl 2-[(1-aza-2-methyl-3-oxobut-1-enyl)methylamino]acetate was added thereto in a dropwise manner over 2.0 hours. Subsequently, the reaction was performed at 40°C for 2 hours. After completing the reaction, the reaction liquid was cooled to 20°C and neutralized with 107.4 g (1.03 mol) of hydrochloric acid. 345.1 g of toluene and 264.8 g of water were added thereto, followed by stirring. After removing the aqueous layer, the toluene layer was washed with water. The toluene layer, from which the washing water had been removed, contained 11.7% by weight of methyl 1,3,4-trimethylpyrazole-5-carboxylate. The yield based on methyl 2-[(1-aza-2-methyl-3-oxobut-1-enyl)methylamino]acetate used was 25.5%. The yield of 1,3,4-trimethylpyrazole-5-carboxylic acid as a hydrolysate was 45.3%.

### Comparative Example 4

In a 500 mL four-neck flask, 93.4 g (0.48 mol) of a 28% sodium methoxide methanol solution and 35.2 g of methanol MeOH were charged, to which 24.4 g (0.22 mol) of calcium chloride was added under stirring. Thereafter, the reaction liquid was heated to 40°C, to which 51.4 g (0.22 mol) of methyl 2-[(1-aza-2-methyl-3-oxobut-1-enyl)methylamino]acetate was added in a dropwise manner over 4.0 hours. Subsequently, the reaction was performed at 40°C for 5 hours. After completing the reaction, the yield of methyl 1,3,4-trimethylpyrazole-5-carboxylate based on methyl 2-[(1-aza-2-methyl-3-oxobut-1-enyl)methylamino]acetate was 61.4%. While 1,3,4-trimethylpyrazole-5-carboxylic acid as a hydrolysate was formed only slightly (0.1% or less), methyl 2-[(1-aza-2-methyl-3-oxobut-1-enyl)methylamino]acetate as the raw material remained in 5.2% presumably because the reaction did not proceed due to the influence of calcium chloride as the dehydration agent.

### Comparative Example 5

In a 300 mL four-neck flask, 14.0 g (0.07 mol) of methyl 2-[(1-aza-2-methyl-3-oxobut-1-enyl)methylamino]acetate and 44.9 g of methanol were charged, to which 16.2 g (0.08 mol) of sodium methoxide and 9.9 g (0.07 mol) of sodium sulfate were added under stirring. Thereafter, the reaction liquid was heated to 40°C, and subsequently reacted at 40°C for 4 hours. After completing the reaction, the yield of methyl 1,3,4-trimethylpyrazole-5-carboxylate based on methyl 2-[(1-aza-2-methyl-3-oxobut-1-enyl)methylamino]acetate was 54.3%. The yield of 1,3,4-trimethylpyrazole-5-carboxylic acid as a hydrolysate was 23.0%.

The above Examples and Comparative Examples reveal the following.

Specifically, as in Comparative Example 1, in the cyclization reaction involving dehydration condensation of a carboxylic acid ester derivative, as the cyclized product has a carboxylic acid ester, water by-produced in the reaction hydrolyzes the carboxylic acid ester to form a carboxylic acid, and therefore the yield of the target carboxylic acid ester is decreased.

As in Examples 1 and 2, on the other hand, in the reaction using a carbonic acid ester compound as a dehydration agent, as the carbonic acid ester compound consumes the by-product water through hydrolyzation thereof, the carboxylic acid ester as the cyclized product is not hydrolyzed but remains as the carboxylic acid ester, and the target cyclized product is obtained with a high yield. The amount of the carboxylic acid ester as the cyclized product that is hydrolyzed to the carboxylic acid in Comparative Example 1 is significantly larger than in Examples 1 and 2, from which it is eventually understood that the yield of the target cyclized product is significantly low.

As in Comparative Example 2, on the other hand, even though water in the system is removed in advance before performing the cyclization reaction, no effect is obtained by the dehydration, and the resulting cyclized product contains a large amount of the carboxylic acid as the hydrolysate of the carboxylic acid ester, from which it is understood that the yield of the target cyclized product having the carboxylic acid ester is small.

As in Comparative Examples 3, 4, and 5, even though an organic dehydration agent or an inorganic solid dehydration agent other than the carbonic acid ester compound is used in the cyclization reaction, no large enhancement of the yield is achieved resulting in a low yield, the effect of dehydration is insufficient, and the cyclized product contains a large amount of the carboxylic acid as the hydrolysate of the carboxylic acid ester.

It is understood from the above that the use of the carbonic acid ester compound as the dehydration agent allows the cyclization reaction to proceed sufficiently, and simultaneously prevents the ester group of the resulting cyclized product from being hydrolyzed to the carboxylic acid, thereby providing the cyclized compound having an ester group (1,3,4-trimethylpyrazole-3-carboxylic acid ester) with a high yield.

### Industrial Applicability

According to the present invention, in the production of a 1,3,4-substituted pyrazole-5-carboxylic acid ester compound, which is used as a medical and agricultural intermediate, the removal of water by-produced during the reaction is important for completing the reaction and providing the target product with a high yield. The use of a carbonic acid ester compound as a dehydration agent enables a convenient dehydration operation, and can provide the target product with a high yield. In the case where an inorganic solid dehydration agent is used for removing water by-produced during the reaction, it is necessary to perform a process of separating the dehydration agent by filtration or the like after completing the reaction, which requires a lot of effort and time for mass production. According to the method of the present invention, on the other hand, an industrial production method that is convenient and is excellent in economic efficiency can be provided owing to the unnecessity of the complicated operation causing a problem, and therefore the present invention has large significance in the medical and agricultural fields.

## Claims

1. A method for producing a cyclized product of a carboxylic acid ester derivative, comprising using a carbonic acid ester compound as a dehydration agent, in a cyclization reaction involving dehydration condensation of a carboxylic acid ester derivative.

2. A method for producing a pyrazole carboxylic acid ester compound, comprising using a carbonic acid ester compound as a dehydration agent, in a formation reaction of a pyrazole ring through a cyclization reaction involving dehydration condensation of a hydrazinocarboxylic acid ester derivative.

3. A method for producing a 1,3,4-substituted pyrazole-5-carboxylic acid ester compound represented by the general formula (2): 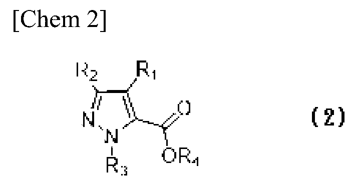 in which R₁ to R₃ each represents a hydrogen atom or an alkyl group having 1 to 4 carbon atoms, and R₄ represents an alkyl group having 1 to 4 carbon atoms, comprising using a carbonic acid ester compound as a dehydration agent, in a cyclization reaction of a hydrazinoacetic acid ester derivative represented by the general formula (1): 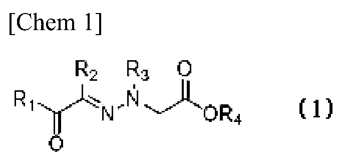 in which R₁ to R₄ have the same meanings as above.

4. The method for producing a cyclized product according to claim 1, wherein in the cyclization reaction involving dehydration condensation of a carboxylic acid ester derivative, the carbonic acid ester is used in an equivalent of 0.5 to 10 equivalents.

5. The method for producing a cyclized product according to claim 1, wherein in the cyclization reaction involving dehydration condensation of a carboxylic acid ester derivative, the carbonic acid ester compound is used in the presence of a base.

6. The method for producing a cyclized product according to claim 5, wherein in the cyclization reaction involving dehydration condensation of a carboxylic acid ester derivative, the base is used in an equivalent of 0.5 to 5 equivalents.

7. The method for producing a cyclized product according to claim 1, wherein the cyclization reaction involving dehydration condensation of a carboxylic acid ester derivative is performed at a reaction temperature of -10 to 130°C.
